# EUROPEAN PATENT SPECIFICATION

(11) **EP 1 813 225 B1**
(45) Date of publication and mention of the grant of the patent: **19.10.2011**
(21) Application number: 07100957.5
(22) Date of filing: 23.01.2007
(51) Int. Cl.: A61F 2/08

(54) **Anchor for tendons used in the reconstruction of a ligament, particularly of the cruciate ligament of the knee**
Verankerung von Sehnen zur Verwendung bei der Rekonstruktion eines Bandes, insbesondere des Kreuzbandes des Knies
Dispositif d'ancrage de tendons pour la reconstruction d'un ligament, en particulier le ligament croisé du genou

(30) Priority: 27.01.2006 IT BO20060007 U
(43) Date of publication of application: 01.08.2007
(73) Proprietor: CITIEFFE S.r.l., 40012 Calderara di Reno (Bologna) (IT)
(72) Inventor: Mingozzi, Franco, BO 40012, Calderara di Reno (IT); Dovesi, Alan, 40138, Bologna (IT)
(74) Representative: Modiano, Micaela Nadia

(56) References cited:
- EP-A- 0 860 146
- GB-A- 2 288 739
- JP-A- 2001 025 478
- US-A1- 2001 025 181

## Description

The present invention relates to an anchor for tendons used in the reconstruction of a ligament, particularly of the cruciate ligament of the knee.

As is known, the cruciate ligament of the knee comprises two bundles: the anteromedial bundle and the posterolateral bundle.

The technique for reconstruction of the anterior cruciate ligament of the knee, known for example from EP-860,146, entails providing a bore which passes through the tibia, in alignment with a bore which passes through the femoral condyle. Two tendons, known as gracilis and semitendinosus, are inserted through the inlet of the tibial bore. Once these two tendons have exited from the opening of the femoral bore, they are passed through an anchor and reinserted first through the femoral bore and then through the tibial bore so that they can be pulled and anchored to the tibial cortex for example by means of one or more staples.

In order to allow adequate abutment of the anchor on the femoral cortex, the anchor is constituted by a cylindrical body, which is inserted in the femoral bore and is provided with an eye for the passage of the two tendons and with a flange for retention on the femoral cortex.

The known anchor has the substantial drawback that since the surface of the cortex is inclined with respect to the axis of the bore in which the cylindrical body of the anchor is to be inserted, the flange cannot rest fully on the surface of the cortex. Indeed, by resting against the cortex only with a small angular portion of the perimetric rim, it assumes an inclined and unstable arrangement which can compromise the subsequent step of tendon tensioning.

US 2001/025181 A1 discloses an orthopedic device including a split-nut fastener having two halves and sliding down a threaded rod until a ring surrounding the two halves forces them together, whereupon the split-nut is threaded tightly onto the threaded rod. A pivot wing bolt assembly is used to secure one end of a threaded rod onto the hollow or marrow of a fractured bone, and together with the split-nut, the pivot wing bolt fixates the fracture bone.

The aim of the present invention is to provide an anchor which allows to obviate the above-mentioned drawbacks.

Within this aim, an object of the present invention is to provide an anchor which is capable of facilitating the operating technique for its installation.

In accordance with the invention, there is provided an anchor for tendons used in the reconstruction of ligaments, as defined in the appended claims.

Further characteristics and advantages of the anchor according to the invention will become better apparent from the following detailed description of an embodiment thereof, illustrated only by way of non-limiting example in the accompanying drawings, wherein:
Figure 1 is a perspective view of an anchor according to the invention;
Figure 2 is a side view of the anchor of Figure 1;
Figure 3 is a side view, rotated through 90° with respect to Figure 2, in which the ring is separated from the cylindrical body;
Figure 4 is a view taken along the line IV-IV of Figure 2;
Figure 5 is a perspective view of the anchor, which illustrates the method of engagement of the tendons;
Figure 6 is a view of the anchor installed for reconstruction of the anterior cruciate ligament of the knee.

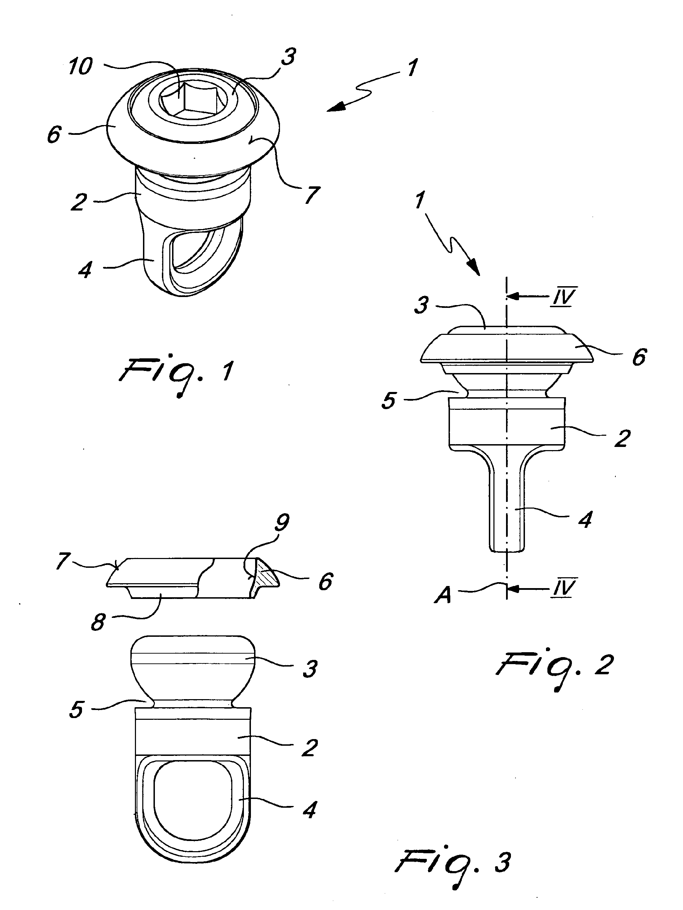

With reference to Figures 1-5, an anchor according to the invention is generally designated by the reference numeral 1 and comprises a substantially cylindrical body 2 which has an axis A and is provided, at one end, with a collar 3 and, at the opposite end, with an eye 4.

The collar 3 is constituted by a substantially hemispherical head, which forms, together with the body 2, an annular groove 5.

The eye 4 lies on a diametrical plane which passes through the axis A of the body 1 and has a substantially ellipsoidal cross-section.

A ring 6 is coupled to the head 3 and forms with it a flange for the abutment of the anchor 1 on the cortex of the femoral condyle of the knee.

In particular, the ring 6 has an upper face 7 which is cambered and an internal face which is integrated with the face of a lower annular lip 8, forming a concave internal surface 9 which is spherically complementary to the surface of the head 3. In this manner, the ring 6 and the head 3 form a spherical coupling which allows the ring 6 to assume an inclined position with respect to the axis A until the annular lip 8 engages in the groove 5 although it remains engaged with the head 3. Conveniently, coupling is provided by inserting with interference the head 3 in the cavity formed by the concave surface 9 so that the ring 6 is retained on the head 3.

The described anchor is completed by a hexagonal recess 10, which is provided axially and centrally in the head 3. The recess 10 is designed for the engagement of a tool, for example a hex key, with which the anchor 1, after being installed in the accommodation seats the bone, can be turned in order to tension the tendons.

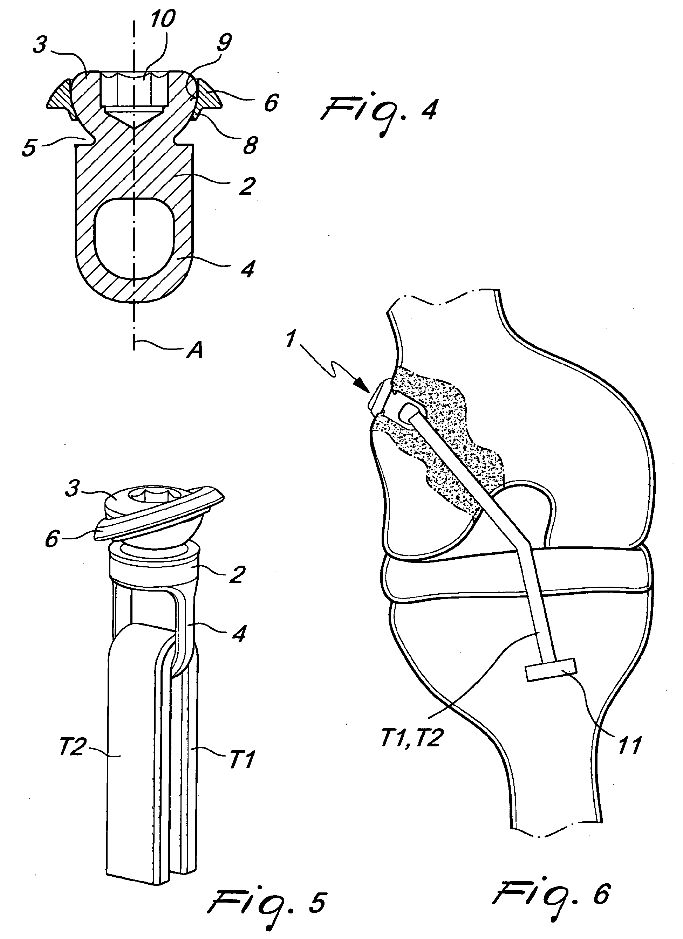

The method for installing the described anchor can be deduced easily from Figure 6, which illustrates the reconstruction of the anterior cruciate ligament of the knee with the known method which uses the double anteromedial and posterolateral bundle, using the semitendinosus and gracilis tendons T1, T2, which are engaged on one side to the respective anchor 1 and are fixed on the opposite side by means of staples 11.

As can be seen, the described invention perfectly achieves the intended aim and object. It is significantly important that the ring 6, once the tension of the tendons has been adjusted to the chosen value, rests perfectly on the femoral cortex, so as to avoid load stresses in highly localized regions which might cause failures of the bone and compromise the preset tensioning of the tendons.

Numerous modifications and variations are possible in the practical embodiment of the invention, all of which are within the scope of the appended claims. Thus, for example, the anchor can be inserted for the same purpose in the tibial bore on its own or in combination with the anchor inserted in the femoral bore.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. An anchor for tendons used in the reconstruction of a ligament, particularly of the cruciate ligament of the knee, comprising a substantially cylindrical body (2) which can be inserted in a bore formed in the femoral condyle or in the tibia and is provided, at one end, with a flange (3, 6) for abutment against the region of the cortex that surrounds the inlet of the bore in which said body has been inserted and, at the opposite end, with an eye (4) for engagement of the tendons (T1, T2) for reconstructing the ligament, which are guided through said bore, said flange being constituted by a head (3) which is rigidly coupled to said cylindrical body (2) and by an abutment ring (6) which is articulated to said head (3) so as to provide a spherical coupling which allows said ring (6) to rest flatly against the region of the cortex that surrounds the inlet of the bore for accommodating said body (2), said head (3) being substantially hemispherical and said ring (6) being coupled thereto, said ring having an internal surface (9) which is concave and complementary to the hemispherical surface of said head (3), **characterized in that** said ring (6) has a cambered upper face (7) and an internal face which is integrated with the face of a lower annular lip (8), which is rigidly coupled to said ring (6) so as to form said concave internal surface (9), which is spherically complementary to the surface of said head (3).

2. The anchor according to claim 1, **characterized in that** said spherical head (3) forms, together with said body (2), an annular groove (5) which can be engaged by said lip (8).

3. The anchor according to claim 1, **characterized in that** said eye (4) lies on a diametrical plane which passes through the axis (A) of said body (2) and has a substantially ellipsoidal cross-section.

4. The anchor according to claim 1, **characterized in that** said spherical coupling is provided by inserting with interference the head (3) in the cavity formed by the concave surface (9), so that the ring (6) is retained articulately on the head (3).

## Patentansprüche

1. Eine Verankerung von Sehnen zur Verwendung bei der Rekonstruktion eines Bandes, insbesondere des Kreuzbandes des Knies, die einen im Wesentlichen zylindrischen Körper (2) umfasst, welcher in eine Bohrung eingesetzt werden kann, die im femoralen Kondylus oder in der Tibia geformt ist, und an einem Ende mit einem Flansch (3, 6) zum Anstoßen gegen den Bereich des Kortex ausgestattet ist, der den Eingang der Bohrung umgibt, in welche der Körper eingesetzt wurde, und am gegenüberliegenden Ende mit einer Öse (4) zum Halten der Sehnen (T1, T2) zur Rekonstruktion des Bandes, die durch die Bohrung geführt werden, wobei der Flansch aus einem Kopf (3) besteht, der starr mit dem zylindrischen Körper (2) gekoppelt ist, und einem Widerlagerring (6), der gelenkig mit dem Kopf (3) verbunden ist und so eine kugelförmige Kopplung bereitstellt, die es dem Ring (6) ermöglicht, flach gegen den Bereich des Kortex anzuliegen, welcher den Eingang der Bohrung zur Aufnahme des Körpers (2) umgibt, wobei der Kopf (3) im Wesentlichen halbkugelförmig ist und der Ring (6) damit verbunden ist, wobei der Ring eine innere Oberfläche (9) hat, die konkav und zur halbkugelförmigen Oberfläche des Kopfes (3) komplementär ist, **dadurch gekennzeichnet, dass** der Ring (6) eine gewölbte obere Fläche (7) und eine innere Fläche hat, welche in die Fläche einer unteren ringförmigen Lippe (8) integriert ist, die starr mit dem Ring (6) gekoppelt ist und so die konkave innere Oberfläche (9) bildet, welche kugelförmig komplementär zur Oberfläche des Kopfes (3) ist.

2. Die Verankerung gemäß Anspruch 1, **dadurch gekennzeichnet, dass** der kugelförmige Kopf (3), gemeinsam mit dem Körper (2), eine Ringnut (5) bildet, in welche die Lippe (8) eingreifen kann.

3. Die Verankerung gemäß Anspruch 1, **dadurch** gekennzeichet, dass die Öse (4) auf einer diametralen Ebene liegt, die durch die Achse (A) des Körpers (2) verläuft und einen im Wesentlichen ellipsenförmigen Querschnitt hat.

4. Die Verankerung gemäß Anspruch 1, **dadurch gekennzeichnet, dass** die kugelförmige Kopplung hergestellt wird durch Einsetzen, mit Eingriff, des Kopfes (3) in den Hohlraum, der von der konkaven Oberfläche (9) gebildet wird, so dass der Ring (6) gelenkig auf dem Kopf (3) gehalten wird.

## Revendications

1. Dispositif d'ancrage pour tendons, utilisé pour la reconstruction d'un ligament, en particulier le ligament croisé du genou, comprenant un corps sensiblement cylindrique (2) qui peut être inséré dans un orifice formé dans le condyle fémoral ou dans le tibia et qui est doté, à une extrémité, d'un rebord (3, 6) conçu pour venir en butée contre la région du cortex qui entoure l'entrée de l'orifice dans lequel ledit corps a été inséré et, à l'extrémité opposée, d'un chas (4) pour engager les tendons (T1, T2) en vue de reconstruire le ligament, qui sont guidés à travers ledit orifice, ledit rebord étant constituée d'une tête (3) qui est couplée de manière rigide audit corps cylindrique (2) et d'une bague de butée (6) qui est articulée sur ladite tête (3) de manière à mettre en oeuvre un couplage sphérique qui permette à ladite bague (6) de reposer à plat contre la région du cortex qui entoure l'entrée de l'orifice destiné à recevoir ledit corps (2), ladite tête (3) étant sensiblement hémisphérique et ladite bague (6) étant couplée à celle-ci, ladite bague ayant une surface interne (9) qui est concave et complémentaire de la surface hémisphérique de ladite tête (3), **caractérisé en ce que** ladite bague (6) a une face supérieure bombée (7) et une face interne qui fait partie de la face d'une lèvre annulaire inférieure (8), qui est couplée de manière rigide à ladite bague (6) de manière à former ladite surface interne concave (9), qui est sphériquement complémentaire de la surface de ladite tête (3).

2. Dispositif d'ancrage selon la revendication 1, **caractérisé en ce que** ladite tête sphérique (3) forme, conjointement avec ledit corps (2), une rainure annulaire (5) sur laquelle peut s'engager ladite lèvre (8).

3. Dispositif d'ancrage selon la revendication 1, **caractérisé en ce que** ledit chas (4) repose selon un plan diamétral passant à travers l'axe (A) dudit corps (2) et présente une coupe transversale sensiblement ellipsoïdale.

4. Dispositif d'ancrage selon la revendication 1, **caractérisé en ce que** ledit couplage sphérique est mis en oeuvre en insérant par serrage la tête (3) dans la cavité formée par la surface concave (9), de sorte que la bague (6) soit retenue sous forme articulée sur la tête (3).
